# EUROPÄISCHE PATENTSCHRIFT

(11) **EP 3 445 865 B1**
(45) Veröffentlichungstag und Bekanntmachung des Hinweises auf die Patenterteilung: **20.04.2022**
(21) Anmeldenummer: 17716867.1
(22) Anmeldetag: 10.04.2017
(51) Int. Cl.: C12P 19/04, C08B 37/00

(54) **VERFAHREN ZUR GEWINNUNG VON ZUMINDEST EINER ODER MEHRERER BETA-GLUCAN-VERBINDUNGEN ODER EINER BETA-GLUCANHALTIGEN FESTSTOFFSUSPENSION AUS HEFEZELLEN**
METHOD FOR OBTAINING AT LEAST ONE OR MORE BETA-GLUCAN COMPOUNDS OR A SOLIDS SUSPENSION CONTAING BETA GLUCAN FROM YEAST CELLS
PROCÉDÉ PERMETTANT D'OBTENIR AU MOINS UN OU PLUSIEURS COMPOSÉS BÊTA-GLUCANE OU UNE SUSPENSION DE SOLIDES CONTENANT DU BÊTA-GLUCANE À PARTIR DE CELLULES DE LEVURE

(30) Priorität: 18.04.2016 DE 102016107140
(43) Veröffentlichungstag der Anmeldung: 27.02.2019
(73) Patentinhaber: GEA Mechanical Equipment GmbH, 59302 Oelde (DE)
(72) Erfinder: HRUSCHKA, Steffen, 59302 Oelde (DE); WEINEKÖTTER, Joachim, 59302 Oelde (DE)
(74) Vertreter: Specht, Peter
(86) Internationale Anmeldenummer: PCT/EP2017/058534
(87) Internationale Veröffentlichungsnummer: WO 2017/182313

(56) Entgegenhaltungen:
- EP-A1- 1 990 419
- WO-A1-97/02356
- DE-A1- 19 835 767
- US-A1- 2012 236 678
- LIU HONGZHI ET AL: "Effects of microfluidization with ionic liquids on the solubilization and structure of [beta]-d-glucan", INTERNATIONAL JOURNAL OF BIOLOGICAL MACROMOLECULES, ELSEVIER BV, NL, Bd. 84, 12. Dezember 2015 (2015-12-12), Seiten 394-401, XP029387801, ISSN: 0141-8130, DOI: 10.1016/J.IJBIOMAC.2015.12.014
- BENITO-ROMÁN Ó ET AL: "Ultrasound-assisted extraction of [beta]-glucans from barley", LWT- FOOD SCIENCE AND TECHNOLOGY, Bd. 50, Nr. 1, 2013, Seiten 57-63, XP028939677, ISSN: 0023-6438, DOI: 10.1016/J.LWT.2012.07.006
- Marc Kellens: "Nano cavitation: a proven new concept", www.oilsandfatsinternational.com, August 2012 (2012-08), Seiten 1-2, XP055385208, Gefunden im Internet: URL:http://www.ctinanotech.com/media/9b55d b3c-fb67-46ae-8d4a-17e36e2a977a/Nano-Cavit ation-a-Proven-New-Concept.pdf [gefunden am 2017-06-26]
- LIU DAN ET AL: "Yeast cell disruption strategies for recovery of intracellular bio-active compounds - A review", INNOVATIVE FOOD SCIENCE AND EMERGING TECHNOLOGIES, ELSEVIER, AMSTERDAM, NL, Bd. 36, 28. Juni 2016 (2016-06-28), Seiten 181-192, XP029676632, ISSN: 1466-8564, DOI: 10.1016/J.IFSET.2016.06.017

## Beschreibung

Die vorliegende Erfindung betrifft ein Verfahren zur Gewinnung von zumindest einer oder mehrerer beta-Glucan-Verbindungen oder einer beta-Glucanhaltigen Feststoffsuspension aus Hefezellen.

Beta-Glucane können in vielerlei Hinsicht sinnvoll genutzt werden. Daher rückt deren industrielle Gewinnung zunehmend in den Fokus von verschiedenen Industriezweigen, beispielsweise der Futtermittel- und Arzneimittelindustrie.

Eine mögliche Gewinnung aus Haferkleie wurde von Michael Urs Beer in seiner Dissertation 1994 mit dem Titel "Gewinnung einer β-Glucan-reichen Haferkleiefraktion und deren Einfluss auf den Cholesterinspiegel in Blut des Menschen" an der ETH Zürich beschrieben.

Die WO 2008/138559 A1 offenbart ein Verfahren zur Isolierung von Glucan aus Hefe unter Zuhilfenahme von Ultraschall. Dabei wird Heißdampf genutzt, um Hefezellen zunächst stärker aufzulösen. Die Hefezellen bleiben bei der Anwendung von Ultraschallwellen erhalten. Der Vacuoleninhalt wird durch die Zellwand hindurch extrahiert. Dabei können Kavitationseffekte entstehen, allerdings führen diese zur Behinderung des Stofftransportes durch die Zellwand durch die Gasbildung an der Zellwand, weshalb eine Temperatur zur Verringerung des Energieeintrags möglichst niedrig gehalten werden sollte, um Kavitationseffekte möglichst zu verringern. Das Auftreten von Kavitationseffekten ist somit ein unerwünschter Effekt, welcher eine Extraktion durch die Zellwand hindurch verhindert.

Die DE 696 30 455 T2 offenbart ebenfalls eine Ultraschallbehandlung zur Extraktion von Hefezell-Inhaltsstoffen aus der Hefezelle.

In der DE 198 35 767 A1 wird ein Verfahren beschrieben, bei welchem Hefezellen zunächst durch Scherung mechanisch zerstört werden, anschließend erfolgt eine Waschung und Gefriertrocknung und schließlich ein enzymatischer Aufschluss unter Gewinnung von beta-Glucanen als Feststofffraktion.

Aus der US 2012/236678 A1 ist der Aufbau und die Funktionsweise eines Nanokavitators bekannt.

Es ist nunmehr die Aufgabe der vorliegenden Erfindung, ein Verfahren zur Gewinnung bereitzustellen, welches einen anderen Weg zur Gewinnung von beta-Glucanen insbesondere mit höheren Ausbeuten und unter Einsparungen von Chemikalien, beschreitet.

Die vorliegende Erfindung löst diese Aufgabe durch ein Verfahren mit den Merkmalen des Anspruchs 1.

Ein erfindungsgemäßes Verfahren zur Gewinnung von einer oder mehrerer beta-Glucan-Verbindungen aus Hefezellen, ist gekennzeichnet durch die folgenden Schritte:
A. Anreicherung von Hefezellen, insbesondere durch Vermehrung in einer Zuckerlösung;
B. Bildung einer beta-glucanhaltigen Hefesuspension umfassend Bestandteile der in Schritt A) angereicherten Hefezellen;
C. Zumindest einmaliges Behandeln der Hefesuspension von Schritt B in zumindest einem Nanokavitator (70, 70', 70" und/oder 70‴) und
D. Abtrennen der beta-Glucan Verbindung oder der beta-Glucan-Verbindungen (90) als Feststoff oder einer beta-glucanhaltigen Feststoffsuspension aus der Hefesuspension von Schritt C.

Mit dem erfindungsgemäßen Verfahren kann somit zumindest eine Verbindung eines beta-Glucans gewonnen werden oder aber auch ein Gemisch aus mehreren unterschiedlichen beta-Glucan-Verbindungen.

Alternativ kann auch eine beta-Glucanhaltigen Feststoffsuspension als Folge einer Extraktabtrennung, z.B. eines Hefeextraktes oder eines Waschextraktes, ermöglicht werden. Der beta-Glucangehalt der Feststoffsuspension wird umso größer sein je höher der Extraktanteil im abgetrennten Extrakt ist.

Der Nanokavitator muss folglich nicht zwingend am Ende des Gewinnungsverfahrens eingesetzt werden, sondern kann an verschiedenen Verfahrensstufen des Gewinnungsverfahrens verwendet werden. Es können auch mehrere Nanokavitatoren im Gewinnungsverfahren genutzt werden oder ein Nanokavitatoren zur Bearbeitung von beta-Glucanhaltigen Hefesuspension welche bei verschiedenen Verfahrensschritten des Gewinnungsverfahrens anfallen.

Die Hefesuspension in Schritt B ist bevorzugt als eine Suspension und/oder Dispersion mit Feststoffen, insbesondere mit Hefebestandteilen, die sowohl nativ, denaturiert oder zumindest teilweise zerstört vorliegen, zu verstehen.

Die Anreicherung von Hefezellen bzw. Hefepilzzellen kann bevorzugt zur Hefezellenzucht Zuckerwasser eingesetzt werden.

Sodann kann eine Hefesuspension gebildet werden, welche zumindest Bestandteile oder ganze Hefezellen aufweist, die zuvor gewonnen wurden. Die Hefezellen können bereits gesterzelt bzw. autolysiert sein. Die Hefesuspension kann auf verschiedene Weise bereitgestellt werden. Es kann sich beispielsweise direkt um die sogenannte Fermentationsbrühe handeln. Bevorzugt jedoch kann die Bildung der Hefesuspension durch Abtrennen der Zuckerlösung und nach erfolgter Autolyse der Hefezellen durch Zugabe einer wässrigen Lauge erfolgen.

Als Hefesuspension umfassend zumindest Bestandteile der nach Schritt A. gewonnenen Hefezellen kommen im Rahmen der vorliegenden Erfindung somit mehrere hefehaltige Suspensionen in Betracht, welche im Verlauf der Verarbeitung der Hefezellen anfallen können. Besonders bevorzugt kann es sich bei der Hefesuspension um eine alkalische wässrige Hefesuspension handeln, welche durch vorhergehende Autolyse und Waschung bereits von einer Vielzahl von Begleitstoffen befreit wurde und in welcher in weit-überwiegendem Anteil tote bzw. autolysierte Hefezellen vorliegen, welche ggf. auch schon teilweise in ihre Bestandteile aufgebrochen sind, so dass nur noch Zellwandfragmente vorliegen.

Anschließend wird die Hefesuspension mit einem Nanokavitator behandelt. Im Unterschied zu einer Scherung der Zellen, erfolgt im Nanokavitator ein teilweises Verdampfen des Lösungsmittels, vorzugsweise von Wasser, so dass sich abzutrennende lösliche Bestandteile der Hefezelle, insbesondere der Hefezellenwandung, deutlich besser in dem Lösungsmittel lösen. Zudem ändert sich die innere Struktur der Hefezellen, so dass Begleitstoffe und Inhaltsstoffe der Hefezelle leichter an das Lösungsmittel abgegeben werden können.

Weitere vorteilhafte Ausgestaltungsvarianten der Erfindung sind Gegenstand der Unteransprüche.

Es ist von Vorteil, wenn die zu behandelnde Hefesuspension in Schritt C eine alkalische Hefesuspension ist mit einem pH-Wert welcher gleich oder größer ist als pH=11. Die alkalische Hefesuspension kann insbesondere eine wässrige Hefesuspension sein. Der pH-Wert kann vorzugsweise einen pH-Wert von pH=12 oder höher aufweisen.

Die Gewinnung der beta-Glucan Verbindung oder der beta-Glucan-Verbindungen als Feststoff oder der beta-Glucanhaltigen Feststoffsuspension aus der Hefesuspension kann vorteilhaft durch Filtration mit einer Filtrationsvorrichtung und/oder Separation im Zentrifugalfeld eines Separators erfolgen.

Die Feststoffsuspension im Rahmen der vorliegenden Erfindung ist vorzugsweise eine wässrige Feststoffsuspension. Im Rahmen der vorliegenden Erfindung sind auch feuchte Feststoffe als Feststoffsuspension zu verstehen.

Die Hefesuspension ist bevorzugt als wässrige Suspension ausgebildet.

Es ist von Vorteil, wenn die Gewinnung der beta-Glucan-Verbindung oder der beta-Glucan-Verbindungen als Feststoff oder einer beta-Glucanhaltigen Feststoffsuspension aus der Hefesuspension durch Filtration mit einer Filtrationsvorrichtung und/oder Separation im Zentrifugalfeld eines Separators erfolgt.

Die Bildung der Hefesuspension gemäß Schritt B kann vorteilhaft eine Autolyse der Hefezellen umfassen. Bereits nach der erfolgten Autolyse kann dann der Nanokavitator eingesetzt werden.

Es ist jedoch noch vorteilhafter wenn die Bildung der Hefesuspension gemäß Schritt B nach der Autolyse eine Abtrennung einer ersten Flüssigphase umfassend die Nährlösung und den Zellextrakt der Hefezellen umfasst unter Bildung einer Feststoffphase oder einer zweiten feststoffhaltigen Flüssigphase. Da die erste Flüssigphase sehr viele gelöste Verbindungen aufweist ist es besser diese erst aus dem Verfahren zu entfernen um anschließend mit einem geeigneten Lösungsmittel und ggf. auch unter einem angepassten pH-Wert eine nochmalige Extraktion durchzuführen.

Besonders vorteilhaft kann die Bildung der Hefesuspension gemäß Schritt B daher auch ein einmaliges oder vorzugsweise mehrmaliges Waschen der Feststoffphase und/oder der zweiten feststoffhaltigen Flüssigphase umfassen, um weitere leichtlösliche Verunreinigungen auszuwaschen.

Die Bildung der Hefesuspension kann gemäß Schritt B ein Suspendieren der autolysierten Hefezellen umfassen, wobei die Hefesuspension eine Behandlung mit dem Nanokavitator erfährt und/oder wobei die Behandlung der Hefesuspension durch den Nanokavitator nach der Zugabe alkalischen Lösung, insbesondere einer Lauge, erfolgt.

Sodann kann die Bildung der Hefesuspension gemäß Schritt B ein Suspendieren und/oder Dispergieren der autolysierten Hefezellen, insbesondere nach dem Waschen, in einer wässrigen Lauge unter Ausbildung der wässrigen Lösung umfassen. Diese wässrige Lösung ist alkalisch und löst besonders gut Begleitstoffe aus Hefezellwänden, so dass eine sehr reine beta-Glucanfraktion gewonnen werden kann.

Aus der vorhergehenden Formulierung wird klar, dass die Hefesuspension in Schritt B bereits die Zuckerlösung selbst mit den darin gezüchteten Hefezellen sein kann, es kann allerdings auch ein Feststoff oder die feststoffhaltige Flüssigphase mit oder ohne einem optionalen Waschvorgang mit Lauge versehen und sodann in den Nanokavitator überführt werden.

Zusätzlich zum Behandeln der Hefesuspension in einem Nanokavitator kann ein Behandeln der Hefesuspension vorteilhaft in einem Homogenisator und/oder in einer Mischvorrichtung erfolgen. Bei den letztgenannten Vorrichtungen erfolgt im Unterschied zur Nanokavitation eine Scherung der Hefezellen.

Es ist von Vorteil, wenn das zusätzliche Behandeln der Hefesuspension in dem Homogenisator und/oder in der Mischvorrichtung unmittelbar vor oder nach dem Behandeln der Hefesuspension mit dem Nanokavitator erfolgt. Dadurch wird eine zu starke Agglomeration der Hefezell-Bestandteile verhindert.

Die Temperatur der Hefesuspension kann während des Behandelns im Nanokavitator vorteilhaft zwischen 20 und 90 °C, vorzugsweise zwischen 50 bis 60°C, betragen.

Die Behandlung im Nanokavitator kann vorzugsweise mehrfach durchgeführt werden.

Alternativ oder zusätzlich kann die Hefesuspension vorteilhaft im Kreislauf geführt werden, so dass sie innerhalb eines Zeitintervalls mehrfach durch den Nanokavitator geleitet wird.

Um vorteilhaft eine große Ausbeute an Hefezellen zu erhalten, erfolgt die Anreicherung der Hefezellen in einer Zuckerlösung ohne Sauerstoffabschluss.

Die Prozessparameter der Behandlung mit dem Nanokavitator, insbesondere die Dauer der Behandlung, der Arbeitsdruck und/oder die Temperatur der Hefesuspension, können anhand der granulometrischen Verteilung der Hefebestandteile in der Hefesuspension und/oder dynamischen Viskosität in Abhängigkeit von der Scherrate geregelt und/oder gesteuert werden.

Die Bildung einer Hefesuspension gemäß Schritt B ist nicht auf einen konkreten Verfahrensschritt beschränkt, sondern es kann bei verschiedenen Verfahrensschritten im Gewinnungsverfahren zur Bildung einer entsprechenden beta-Glucanhaltigen bzw. hefezellwandhaltigen Hefesuspension kommen. Die gebildete Hefesuspension gemäß Schritt B. kann vorteilhaft
a) Nach der Anreicherung der Hefezellen und vor der Autolyse
b) Nach der Autolyse und vor dem Separieren des Hefeextraktes
c) Beim Waschen und vor dem Abtrennen des Waschextraktes und/oder
d) Nach der Laugenzugabe
vorliegen und durch den Nanokavitator behandelt werden.

Nachfolgend soll die Erfindung anhand der beiliegenden Figuren und anhand von einer Ausführungsvariante näher erläutert werden. Es zeigen:
- Fig. 1: eine schematische Darstellung des Ablaufs eines erfindungsgemäßen Verfahrens zur Herstellung;
- Fig. 2: ein Diagramm zur granulometrischen Verteilung von alkalischen Hefesuspensionen mit Hefezellbestandteilen vor, während und nach der Nanokavitation; und
- Fig. 3: ein Diagramm der dynamischen Viskosität in Abhängigkeit zur Scherrate bei den alkalischen Hefesuspensionen mit Hefezellbestandteilen der Fig. 2.

Im Stand der Technik ist eine Ultraschallbehandlung von Hefezellen bekannt, welche es ermöglicht eine Extraktion von Zellinhalten durch die Zellwand hindurch durchzuführen. Unterstützend kann hierfür Heißdampf eingesetzt werden um die Durchlässigkeit der Zellwand zu erhöhen und so die Förderung durch die Zellwand hindurch zu erleichtern. Bei der Ultraschallbehandlung kann Kavitation auftreten, welche jedoch die Zellwandoberfläche mit Gas benetzt und daher eher unerwünscht ist. Die Extraktion durch die Zellwand erfolgt dabei bei Flüssigkeitskontakt mit der Zellwand. D.h. die Ultraschallbehandlung unterstützt den Stofftransport und die Kavitation an der Zelloberfläche ist dem hinderlich.

Von der Ultraschallbehandlung unterscheidet sich die Behandlung in einem Nanokavitator deutlich. Die zu behandelnde Suspension wird mit einer Pumpe unter hohem Druck in den Nanokavitator gepresst. Auf Grund der speziellen Strömungsgeometrie entstehen innerhalb des Nanokavitators Bereiche, in denen der Druck der Flüssigkeit soweit abfällt, dass der Siedepunkt der Flüssigkeit unterschritten wird und diese schlagartig verdampft. Die hierbei freiwerdende Energie führt unter anderem zur Beschädigung der Zellwände der in der Suspension befindlichen Hefe.

Nach der Schädigung der Zellwände der Hefezellen kann die Abtrennung von Glucan aus der Hefesuspension anschließend im Zentrifugalfeld und/oder in einem Druckfilter und/oder einer Presse erfolgen. Somit wird der Kavitationseffekt von der Extraktion entkoppelt im Gegensatz zur Ultraschallbehandlung im Stand der Technik, wo beides zeitgleich erfolgt und die Kavitation den Transport behindert. Durch Schädigung der Zellwand kann die Extraktion wesentlich effektiver erfolgen und durch die räumliche Entkopplung beider Prozessschritte kann eine Vorrichtung zur Realisierung des Verfahrens vereinfacht werden

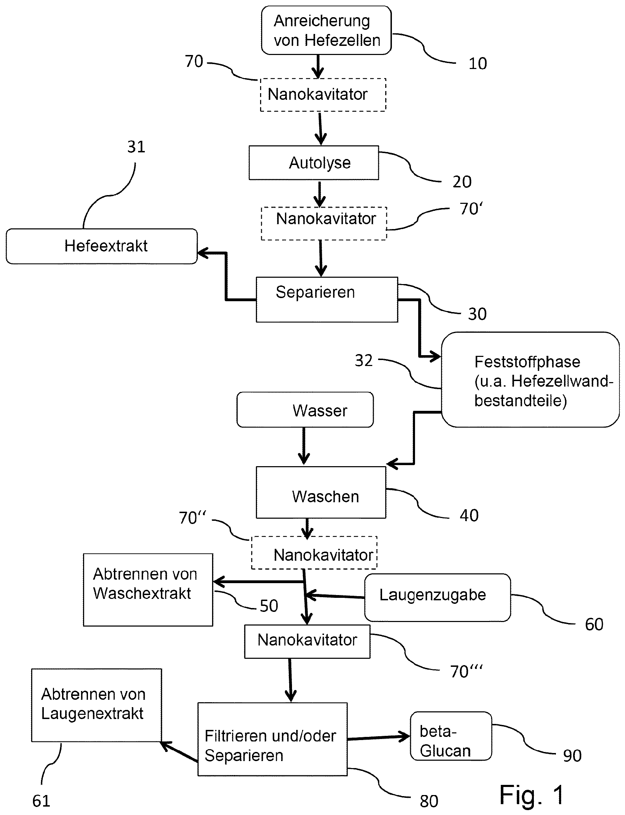

Fig. 1 zeigt beispielhaft einen Verfahrensablauf eines erfindungsgemäßen Verfahrens zur Gewinnung von einer oder mehreren beta-Glucan-Verbindungen aus Hefezellen.

Bei der Stoffklasse der Glucane handelt es um Polysaccharide, bestehend aus Glucose-Monomereinheiten, welche in alternierender Weise über beta-glycosidische 1,6- 1,3 und 1,2 Bindungen verknüpft sind. Sie bilden bekannterweise das Stützgerüst von Hefezellwänden. Beta-Glucan hat positive Eigenschaften auf das Immunsystem und wird daher als möglicher Ersatz oder als Ergänzung zu Antibiotika eingesetzt. Alternativ kann es auch als Nahrungsmittelergänzungsmittel eingesetzt werden um die Sterblichkeitsrate von Lebewesen, insbesondere von Wasserlebewesen, z.B. Schalentieren, Fischen und dergleichen, herabzusetzen. Auch als Tierfutter für Rinder, Schweine und Geflügel können daher beta-Glucane genutzt werden.

Zellwände von Hefen bestehen im Wesenlichen aus beta-Glucanen, Mannan-Zucker-Polymeren, Proteinen, Lipiden und geringen Anteilen an Chitin. Es gilt im Rahmen der vorliegenden Erfindung bei der Gewinnung von beta-Glucanen diese restlichen Verbindungen möglichst vollständig herauszulösen.

Zur Gewinnung von beta-Glucanen aus Hefepilzen nach dem erfindungsgemäßen Verfahren erfolgt in einem ersten Schritt 10 eine Anreicherung von Hefepilze einer oder mehrerer Hefekulturen. Dies erfolgt in einer Hefeanreicherung. Bevorzugt kann Hefeanreicherung nicht als alkoholische Gärung sondern unter Sauerstoffeinfluss in einer Zuckerlösung erfolgen, wobei es zu einer Anreicherung der Hefepilze kommt. Die Anwendung einer Hefepilz-Züchtung während einer alkoholischen Gärung ist demgegenüber weniger ertragreich und folglich weniger bevorzugt. Die Nährlösung mit den gezüchteten Hefezellen ist eine gebildete Hefesuspension, gemäß Schritt B.

Bereits nach der Züchtung kann optional und vorteilhaft eine Behandlung der Hefezellen durch einen Nanokavitator 70 erfolgen. Dabei kommt es zu einer Zerstörung einer Vielzahl von Hefezellen.

Nach der Vermehrung der Hefezellen in einem Zuchttank können diese Hefezellen enzymatisch, thermisch oder mechanisch in einem weiteren Verfahrensschritt 20 durch Autolyse weiterverarbeitet werden. Bei der Autolyse handelt es sich um die enzymatische Selbstauflösung von Hefezellen, welche im Rahmen der vorliegenden Erfindung mechanisch und/oder thermisch unterstützt werden kann. Zudem können noch zusätzlich Enzyme zugegeben werden, welche unter Abtötung oder Unterstützung von hefeeigenen Enzymen eine Autolyse ermöglichen oder unterstützen.

Diese Autolyse geht üblicherweise einher mit einem Absterben eines Großteils der Hefezellen. Die autolysierte Hefesuspension umfasst somit Feststoffe in Form von lebenden und toten Hefezellen, sowie von festen Bruchstücken z.B. der Hefezellwand und dem Hefeextrakt 31.

Diese Hefesuspension kann ebenfalls eine Hefesuspension gemäß Schritt B des erfindungsgemäßen Verfahrens sein und sie kann optional mit einem Nanokavitator 70' behandelt werden.

Sodann kann in einem weiteren Verfahrensschritt ein Separieren bzw. ein Abtrennen 30 der Hefezellen und Hefezellenbestandteile als Feststoffphase 32 von der Zucker- bzw. Nährstofflösung 31 und weiteren löslichen Bestandteilen der Zelle, z.B. dem Zellextrakt oder teilweise auch von lebenden Zellen erfolgen.

Die Separation erfolgt bevorzugt in einem Zentrifugalfeld eines Separators. Alternativ oder zusätzlich kann auch eine Filtration erfolgen. Durch das Separieren 30 erhält man eine proteinhaltige Phase 31 und eine Feststoffphase 32 mit Hefepilzzellen, - zellbruchstücke, toten Hefepilzzellen, Hefepilzzellwänden und lebenden Hefepilzzellen mit einer Dichte die größer ist als Wasser.

Anschließend kann diese Feststoffphase 32 im Rahmen einer Nachbehandlung einem ein- oder mehrstufigen Waschen 40 unterzogen werden, um ggf. auch weitere Begleitstoffe zu gewinnen. Dabei können Lösungsmittel, insbesondere Wasser, zum Einsatz kommen die einerseits den Aufschluss der Zellen unterstützen und andererseits die Mikrobiologische Stabilität sicherstellen sollen. Die hefeeigenen Proteasen und Hydrolasen hydrolysieren dann die Zellinhalte, wodurch Proteine in Peptide und Aminosäuren, DNA und RNA zu Nukleotiden aufgespalten werden. Um einen höheren Nukleotidgehalt zu erzielen, können die hefeeigenen Enzyme durch Zugabe von Nucleasen ergänzt werden. Dies kann unter anderem deswegen wünschenswert sein, weil die Ribonucleotide Guanylsäure und Inosinsäure die geschmacksverstärkende Wirkung des Hefeextraktes vervielfachen.

Bei diesem Waschen kann die Hefesuspension aus Wasser und Hefebestandteilen optional mit einem Nanokavitator 70" behandelt werden.

Um diese Zellbegleitstoffe danach zu gewinnen wird der Waschextrakt 50 von den Zellschalen und den Zellbruchstücken vorzugsweise mit Hilfe von Zentrifugen und/oder Filtern abgetrennt.

Die nach der Abtrennung des Waschextraktes und der Begleitstoffe verbleibende Zellschalenphase enthält wesentliche Mengen an beta-Glucanen, welche durch eine weitere Zerstörung der Zellen in gereinigter Form gewonnen werden können.

Die Zellschalenphase wird in einer alkalischen, vorzugsweise wässrigen, Lösung 60 dispergiert und/oder suspendiert. Der ph-Wert dieser zugesetzten Lösung bzw. der Lauge beträgt vorzugsweise pH=11 oder höher. Bei der Lauge kann es sich bevorzugt um eine zumindest 20%ige Lauge, insbesondere um eine zumindest 30%ige Lauge handeln, wobei sich die Prozentangaben auf Gewichtsprozent von Hydroxid-Salz in Wasser beziehen.

Sodann erfolgt ein optionales Behandeln der dispergierten und/oder suspendierten Zellschalen-phase mit einem Nanokavitator 70 "'. Bei einem solchen Nanokavitator durchströmt das alkalische Fluid mit den suspendierten oder dispergierten Hefezellbestandteilen einen Kanal mit Strömungsstörungen, so dass im Unterschied zu einer Scherung wie sie z.B. in einem Intensivmischer erfolgt, in einem Nanokavitator eine Druckerhöhung und Entspannung in Sekundenbruchteilen mehrmals hintereinander erfolgt.

Basierend auf dem Strömungsprinzip von Bernoulli können partiell hohe Fließ-Geschwindigkeiten mit niedrigen Drucken, bis zu Unterdrücken, in unmittelbarer Abfolge zur geringen Strömungsgeschwindigkeiten mit hohen Drücken bis zu 80 bar erzeugt werden.

Dabei ist die Entspannung so groß, dass bei dem durch die Strömung entstehenden Unterdruck der Lösungsmittelbestandteil des Fluides z.B. Wasser in den gasförmigen Zustand übergeht, bevor es im nächsten Augenblick wieder kondensiert. Dies führt zu einer Veränderung der Hefezellschalen.

Einen entsprechenden Nanokavitator kann man als "nano cavitation reactor" beispielsweise bei der Firma Cavitation Technologies Inc. (CTI) erwerben.

Damit ist überraschenderweise eine noch höhere Extraktionsrate erzielbar als bei mehrmaliger Waschung oder einer Druckerhöhung z.B. bei Einsatz eines Homogenisators.

Der Trockensubstanzgehalt der Feststoffe ändert sich kaum, jedoch ändert sich die Zusammensetzung der Zwickelflüssigkeit, also der kontinuierlich Phase. Die Zwickellösung bzw. Zwickelflüssigkeit ist die Flüssigkeit die zwischen den Hefezellen vorliegt. Sie stellt das Kontinuum in der Suspension dar. Dies ist nur Infolge des Stoffaustausches bezogen auf die Zelle- zwischen Außen und Innen erklärbar und begründet somit die gefundenen höheren Extraktionsraten.

Auch ändert sich das rheologische Verhalten der Suspension. Infolge des mehrmaligen Kavitationseffektes ist eine Viskositätsabnahme zu beobachten.

Es konnte bei der Behandlung durch einen Nanokavitator eine Viskositätsabnahme in der Suspension beobachtet werden, ohne dass optisch Veränderung der Zellen zu erkennen ist. Dies wird daran deutlich, dass die granulometrische Verteilung keine signifikanten Änderung aufzeigt, infolge der Anwendung, wohl aber die Viskosität bei vergleichbaren Scherraten abnimmt.

Das plastisch-dilatante Fließverhalten nach Herschel-Bulkley bleibt somit erhalten, jedoch mit signifikant niedrigeren Viskositäten. Dies wird auf eine Veränderung der suspendierten Zellen zurückgeführt, da der Trockensubstanzgehalt nicht verändert wurde.

Zum Erhalt einer gereinigten beta-Glucanphase wird sodann die Lauge nach der Behandlung in dem Nanokavitator von den suspensierten und/oder dispergierten Feststoffen getrennt. Dies kann durch eine Filtration oder eine Separation im Zentrifugalfeld eines Separators erfolgen.

Die vorgenannte Verfahrensabfolge beischreibt einen bevorzugten Weg der Gewinnung von beta-Glucanen. Es ist allerdings auch möglich beta-Glucan direkt nach der Autolyse durch Zugabe einer Lauge und unter Verwendung eines Nanokavitators zu gewinnen. Die weiteren vorbereitenden Schritte dienen dabei zur besseren Reinheit des Ausgangsstoffes vor dessen Überführung in den Nanokavitator und der Isolation weiterer Wertprodukte.

Es ist beispielsweise auch möglich einen Nanokavitator direkt vor oder bevorzugt nach der Autokatalyse einzusetzen. Bei der Behandlung einer hefehaltigen Hefesuspension mit dem Nanokavitator empfiehlt es sich besonders bevorzugt sofern die Hefesuspension einen alkalischen pH-Wert aufweist, um besser Verunreinigungen in Lösung zu bringen. Somit werden Verunreinigungen aus den nicht-löslichen beta-Glucanen entfernt.

Der Ausgangsstoff vor der Behandlung mit dem Nanokavitator kann folglich eine Hefesuspension, enzymatisch autolysierte Hefesuspension oder gewaschenes Hefesuspensionskonzentrat vor und nach einer pH-Verschiebung sein.

Die Behandlung der Hefesuspension mit dem Nanokavitator erfolgt bevorzugt bei Drücken von 50 bis 150 Bar, vorzugsweise bei 60-90 bar.

Die Temperatur der Hefesuspension mit den Hefebestandteilen bei deren Behandlung im Nanokavitator beträgt 20°C bis 90°C vorzugsweise 50-60°C.

Die Behandlung der Hefesuspension mit den Hefebestandteilen durch den Nanokavitator kann in einem einmaligen oder mehrmaligen Durchlauf erfolgen oder im Kreislauf geführt werden.

Der alkalische Aufschluss der Zelle zur Freisetzung störender Substanzen, letztlich zur Erhöhung der Reinheit des Beta-glucanhaltigen Feststoffs, welcher aus der Hefezellwand gewonnen wurde, wird nach der Nanokavitation deutlich verbessert. Die nötige Lauge dafür wird reduziert auf ca. 20-33% der Laugenmenge ohne Einsatz eines Nanokavitators. Damit verringert sich die Salzbeladung des Beta-Glucans, es hat also eine höhere Reinheit. Des Weiteren verringert sich die Viskosität.

Anschließend wird die Lauge durch Filtration und/oder zentrifugaler Separation 80 abgetrennt. Zurück bleibt ein sehr reines beta-Glucan 90.

Zusätzlich kann zur beta-Glucangewinnung vor oder nach der Behandlung durch den Nanokavitator eine Mischvorrichtung und/oder ein Homogenisator genutzt werden. Insbesondere die alkalische Hefesuspension mit den Hefezellenbestandteilen, insbesondere mit den Zellwandbestandteilen, können in die Mischvorrichtung und/oder den Homogenisator überführt werden um dort ein verbessertes Herauslösen von Bestandteilen aus der Zellwand zu erreichen. Dadurch bleiben beta-Glucane mit hoher Reinheit zurück.

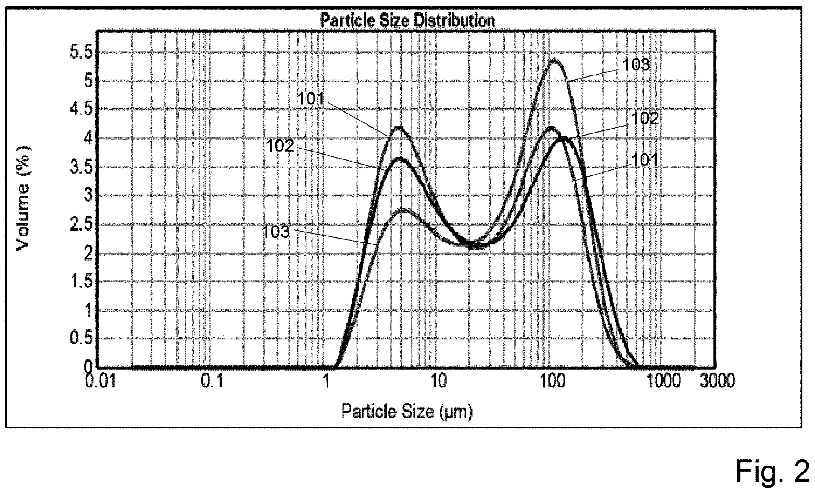

Fig. 2 zeigt eine granulometrische Verteilung 101, in Fig. 2 "particle size distribution" genannt, mit der Partikelgröße bzw. "particle size" in µm bezogen auf das Volumen bzw. "volume" in % in der Hefesuspension) der Hefezellenbestandteile einer alkalischen Hefesuspension vor der Behandlung mit dem Nanokavitator, eine granulometrische Verteilung 102 während der Behandlung mit dem Nanokavitator und eine granulometrische Verteilung 103 nach der Behandlung mit dem Nanokavitator. Man erkennt eine zunehmende Verklumpung bzw. eine Agglomeration der suspendierten Feststoffe innerhalb einer Hefezelleinheit. Allerdings ändert sich dabei die Gesamtgröße der Hefezelleinheit nicht. Dies lässt sich dadurch erklären, dass die Zellen bei einer Verklumpung vollständig zerstört wurden und sich die Zellbestandteile neu zu größeren Einheiten bzw. Agglomeraten umorientieren. Durch diese Zerstörung wird es möglich weitere lösliche Bestandteile in die Lauge bzw. das alkalische Lösungsmittel zu überführen.

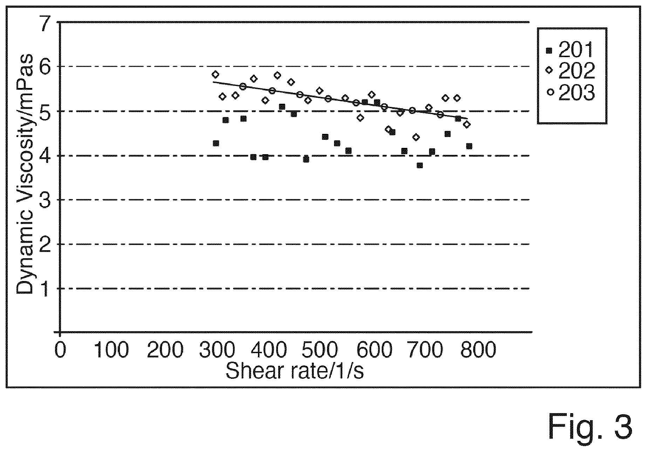

Fig. 3 zeigt ein Diagramm der Scherrate, in Fig. 3 "shear rate" genannt, bezogen auf die dynamische Viskosität, in Fig. 3 "dynamic viscosity" genannt, für die Hefesuspensionen der Fig. 2. Vor der Behandlung durch den Nanokavitator ergibt sich eine gute Korrelation zwischen der Scherrate und der dynamischen Viskosität. Jeder der Messpunkte 203 liegt auf der dargestellten Korrelationsgeraden. Diese Messpunkte 203 beziehen sich auf die alkalische Hefesuspension mit den Hefezellbestandteilen vor deren Behandlung mit dem Nanokavitator. Die Messpunkte 202 beziehen sich auf die alkalische Hefesuspension mit den Hefezellenbestandteilen nach einer anfänglichen Behandlung mit dem Nanokavitator. Man erkennt noch eine gewisse Korrelation der Messpunkte entlang der Korrelationsgeraden. Die Messpunkte 201 beziehen sich auf die alkalische Hefesuspension mit den Hefezellenbestandteilen nach einer vollständigen Behandlung mit dem Nanokavitator. Man erkennt keinerlei Korrelation zwischen der dynamischen Viskosität und der Scherrate.

Die Korrelation kann damit erklärt werden, dass es sich bei einer entsprechenden Hefesuspension und/oder Hefedispersion um ein Fluid handelt dessen Viskosität sich mit höherer Scherrate verringert. Nach der Nanokavitation erfolgt eine deutlich höhere Streuung der Messpunkte bei Änderung der Scherrate. Dies lässt sich dadurch erklären, dass Bruchstücke der Hefezellen bzw. Bruchstücke der Hefezellwandung entstanden sind, welche deutlich feiner verteilt sind. Eine Korrelation zwischen Scherrate und dynamischer Viskosität kann dabei nicht mehr beobachtet werden, so dass die Hefezellen und auch die Hefezellwände durch die Behandlung mit dem Nanokavitator vollständig aufgebrochen wurden.

Hierfür kann bei jedem möglichen Einsatz des Nanokavitators 70, 70', 70", 70‴ die Hefesuspension mehrmals durch den Nanokavitator behandelt werden.

Der Einsatz des Nanokavitators kann an mehreren Stellen des Verfahrens erfolgen. Erfindungsgemäß erfolgt der Einsatz des Nanokavitators jedoch zumindest einmal nach Bildung der Hefesuspension.

### Bezugszeichenliste

- 10: Anreicherung von Hefezellen
- 20: Autolyse
- 30: Separieren
- 31: Hefeextrakt
- 32: Feststoffphase mit festen Hefezellbestandteilen und Hefezellen
- 40: Waschen
- 50: Abtrennen von Waschextrakt
- 51: Waschextrakt
- 60: Laugenzugabe bzw. -reaktion
- 61: Laugenextrakt
- 70, 70', 70", 70‴: Nanokavitator
- 80: Filtrieren und/oder Separieren
- 90: beta-Glucan
- 101-103: Messkurven
- 201-203: Messpunkte

## Patentansprüche

1. Verfahren zur Gewinnung einer oder mehrerer beta-Glucan-Verbindungen oder einer beta-Glucanhaltigen Feststoffsuspension aus Hefezellen, **gekennzeichnet durch** die folgenden Schritte:
A. Anreicherung von Hefezellen;
B. Bildung einer beta-glucanhaltigen Hefesuspension umfassend Bestandteile der in Schritt A) angereicherten Hefezellen;
C. Zumindest einmaliges Behandeln der Hefesuspension von Schritt B in zumindest einem Nanokavitator (70, 70', 70" und/oder 70‴) und
D. Abtrennen der beta-Glucan Verbindung oder der beta-Glucan-Verbindungen (90) als Feststoff oder einer beta-glucanhaltigen Feststoffsuspension aus der Hefesuspension von Schritt C.

2. Verfahren nach Anspruch 1, **dadurch gekennzeichnet, dass** die zu behandelnde Hefesuspension in Schritt C eine alkalische Hefesuspension ist mit einem pH-Wert welcher gleich oder größer ist als pH=11.

3. Verfahren nach Anspruch 1 oder 2, **dadurch gekennzeichnet, dass** die Abtrennung in Schritt D durch Filtration mit einer Filtrationsvorrichtung und/oder Separation im Zentrifugalfeld eines Separators (30, 50 und/oder 80) erfolgt.

4. Verfahren nach einem der vorhergehenden Ansprüche, **dadurch gekennzeichnet, dass** die Bildung der Hefesuspension gemäß Schritt B eine Autolyse (20) der Hefezellen umfasst.

5. Verfahren nach Anspruch 4, **dadurch gekennzeichnet, dass** die Bildung der Hefesuspension gemäß Schritt B nach der Autolyse (20) eine Abtrennung (30) eines Hefeextraktes (31) umfasst unter Bildung einer Feststoffphase (32) oder einer feststoffhaltigen Flüssigphase, wobei die Feststoffphase oder die zweite Flüssigphase feste Bestandteile der Hefezellen aufweist und beta-Glucanhaltig ist.

6. Verfahren nach Anspruch 5, **dadurch gekennzeichnet, dass** die Bildung der Hefesuspension gemäß Schritt B ein einmaliges oder vorzugsweise mehrmaliges Waschen (40) der Feststoffphase und/oder der feststoffhaltigen Flüssigphase umfasst.

7. Verfahren nach einem der vorhergehenden Ansprüche, **dadurch gekennzeichnet, dass** die Bildung der Hefesuspension gemäß Schritt B ein Suspendieren der autolysierten Hefezellen umfasst.

8. Verfahren nach Anspruch 7, **dadurch gekennzeichnet, dass** die Hefesuspension eine Behandlung mit dem Nanokavitator (70, 70' und/oder 70") erfährt und/oder dass die Behandlung der Hefesuspension durch den Nanokavitator (70"') nach der Zugabe einer alkalischen Lösung, insbesondere einer Lauge (60), erfolgt.

9. Verfahren nach einem der vorhergehenden Ansprüche, **dadurch gekennzeichnet, dass** zusätzlich zum Behandeln der Hefesuspension in einem Nanokavitator (70, 70', 70" und/oder 70‴) ein Behandeln der Hefesuspension in einem Homogenisator und/oder in einer Mischvorrichtung erfolgt.

10. Verfahren nach Anspruch 9, **dadurch gekennzeichnet, dass** das zusätzliche Behandeln der Hefesuspension in dem Homogenisator und/oder in der Mischvorrichtung unmittelbar vor oder nach dem Behandeln der Hefesuspension mit dem Nanokavitator (70, 70', 70" und/oder 70‴) erfolgt.

11. Verfahren nach einem der vorhergehenden Ansprüche, **dadurch gekennzeichnet, dass** die Temperatur der Hefesuspension während des Behandelns im Nanokavitator (70, 70', 70" und/oder 70‴) zwischen 20 und 90°C, vorzugsweise zwischen 50 bis 60°C erfolgt

12. Verfahren nach einem der vorhergehenden Ansprüche, **dadurch gekennzeichnet, dass** die Behandlung im Nanokavitator (70, 70', 70" und/oder 70‴) mehrfach durchgeführt wird.

13. Verfahren nach einem der vorhergehenden Ansprüche, **dadurch gekennzeichnet, dass** die Behandlung im Nanokavitator (70, 70', 70" und/oder 70‴) bei Drücken von 50 bis 150 Bar, vorzugsweise bei 60-90 bar, erfolgt.

14. Verfahren nach einem der vorhergehenden Ansprüche, **dadurch gekennzeichnet, dass** die Hefesuspension im Kreislauf geführt wird, so dass sie innerhalb eines Zeitintervalls mehrfach durch den Nanokavitator (70, 70', 70" und/oder 70‴) geleitet wird.

15. Verfahren nach einem der vorhergehenden Ansprüche, **dadurch gekennzeichnet, dass** die Anreicherung der Hefezellen (10) in einer Zuckerlösung ohne Sauerstoffabschluss erfolgt.

16. Verfahren nach einem der vorhergehenden Ansprüche, **dadurch gekennzeichnet, dass** zumindest ein Prozessparameter der Behandlung mit dem Nanokavitator (70, 70', 70" und/oder 70‴), insbesondere die Dauer der Behandlung, der Arbeitsdruck und/oder die Temperatur der Hefesuspension, anhand der granulometrischen Verteilung der Hefebestandteile in der Hefesuspension und/oder dynamischen Viskosität in Abhängigkeit von der Scherrate gesteuert und/oder geregelt wird.

17. Verfahren nach einem der vorhergehenden Ansprüche, **dadurch gekennzeichnet, dass** die gebildete Hefesuspension gemäß Schritt B.
a) Nach der Anreicherung der Hefezellen (10) und vor der Autolyse (20)
b) Nach der Autolyse (20) und vor dem Separieren des Hefeextraktes (31)
c) Beim Waschen( 40) und vor dem Abtrennen (50) des Waschextraktes (51)
und/oder
d) Bei der Laugenreaktion (60) und vor dem Abtrennen (80) des Laugeextraktes (81)
vorliegt und durch den Nanokavitator (70, 70', 70" und/oder 70‴) gemäß Schritt C behandelt wird.

## Claims

1. A method for recovering one or more beta-glucan compounds or a beta-glucan-containing solids suspension from yeast cells, **characterized by** the following steps:
A. enrichment of yeast cells;
B. formation of a yeast suspension, as a beta-glucan-containing yeast suspension, comprising at least constituents of the yeast cells enriched according to step A);
C. treatment of the yeast suspension of step B at least once in at least one nanocavitator (70, 70', 70" and/or 70‴) and
D. removal of the beta-glucan compound or the beta-glucan compounds (90) as solid or of a beta-glucan-containing solids suspension from the yeast suspension of step C.

2. The method as claimed in claim 1, **characterized in that** the yeast suspension to be treated in step C is an alkaline yeast suspension having a pH which is equal to or greater than pH=11.

3. The method as claimed in claim 1 or 2, **characterized in that** the removal in step D is carried out by filtration using a filtration device and/or separation in the centrifugal field of a separator (30, 50 and/or 80).

4. The method as claimed in any of the preceding claims, **characterized in that** the formation of the yeast suspension according to step B encompasses an autolysis (20) of the yeast cells.

5. The method as claimed in claim 4, **characterized in that** the formation of the yeast suspension according to step B encompasses, after the autolysis (20), a removal (30) of a yeast extract (31) to form a solid phase (32) or a solids-containing liquid phase, the solid phase or the second liquid phase comprising solid constituents of the yeast cells and being beta-glucan-containing.

6. The method as claimed in claim 5, **characterized in that** the formation of the yeast suspension according to step B encompasses a washing (40) of the solid phase and/or the solids-containing liquid phase once or preferably multiple times.

7. The method as claimed in any of the preceding claims, **characterized in that** the formation of the yeast suspension according to step B encompasses a suspending of the autolysed yeast cells.

8. The method as claimed in claim 7, **characterized in that** the yeast suspension undergoes a treatment with the nanocavitator (70, 70' and/or 70") and/or **in that** the treatment of the yeast suspension by the nanocavitator (70‴) is carried out after the addition of an alkaline solution, especially an alkali (60).

9. The method as claimed in any of the preceding claims, **characterized in that**, in addition to the treatment of the yeast suspension in a nanocavitator (70, 70', 70" and/or 70‴), a treatment of the yeast suspension is carried out in a homogenizer and/or in a mixing device.

10. The method as claimed in claim 9, **characterized in that** the additional treatment of the yeast suspension in the homogenizer and/or in the mixing device is carried out immediately before or after the treatment of the yeast suspension with the nanocavitator (70, 70', 70" and/or 70‴).

11. The method as claimed in any of the preceding claims, **characterized in that**, during the treatment in the nanocavitator (70, 70', 70" and/or 70‴), the temperature of the yeast suspension is carried out between 20 and 90°C, preferably between 50 to 60°C.

12. The method as claimed in any of the preceding claims, **characterized in that** the treatment in the nanocavitator (70, 70', 70" and/or 70‴) is carried out multiple times.

13. The method as claimed in any of the preceding claims, **characterized in that** the treatment in the nanocavitator (70, 70', 70" and/or 70‴) is carried out at pressures of from 50 to 150 bar, preferably at 60-90 bar.

14. The method as claimed in any of the preceding claims, **characterized in that** the yeast suspension is guided in a loop, with the result that it is conducted multiple times through the nanocavitator (70, 70', 70" and/or 70‴) within one time interval.

15. The method as claimed in any of the preceding claims, **characterized in that** the enrichment of the yeast cells (10) is carried out in a sugar solution without exclusion of oxygen.

16. The method as claimed in any of the preceding claims, **characterized in that** at least one process parameter for the treatment with the nanocavitator (70, 70', 70" and/or 70"'), especially the duration of the treatment, the working pressure and/or the temperature of the yeast suspension, is controlled and/or regulated on the basis of the particle size distribution of the yeast constituents in the yeast suspension and/or dynamic viscosity as a function of the shear rate.

17. The method as claimed in any of the preceding claims, **characterized in that** the yeast suspension formed according to step B is present
a) after the enrichment of the yeast cells (10) and before the autolysis (20)
b) after the autolysis (20) and before the separation of the yeast extract (31)
c) during the washing (40) and before the removal (50) of the wash extract (51)
and/or
d) during the alkali reaction (60) and before the removal (80) of the alkali extract (81)
and is treated according to step C by the nanocavitator (70, 70', 70" and/or 70‴).

## Revendications

1. Procédé pour l'extraction d'un ou plusieurs composés bêtaglucane ou d'une suspension solide contenant des bêtaglucanes de cellules de levure, **caractérisé en ce qu'**il comprend les étapes suivantes :
A. multiplication de cellules de levure ;
B. formation d'une suspension de levure contenant des bêtaglucanes, comprenant des composantes des cellules de levure multipliées dans l'étape A ;
C. traitement au moins une fois de la suspension de levure de l'étape B dans au moins un nanocavitateur (70, 70', 70" et/ou 70‴) et
D. séparation du ou des composés bêtaglucane (90) sous forme solide ou d'une suspension de solides contenant des bêtaglucanes de la suspension de levure de l'étape C.

2. Procédé selon la revendication 1, **caractérisé en ce que** la solution de levure à traiter de l'étape C est une suspension de levure alcaline dont le pH est égal ou supérieur à 11.

3. Procédé selon la revendication 1 ou 2, **caractérisé en ce que** la suspension dans l'étape D est réalisée par filtration dans un dispositif de filtration et/ou par séparation dans le champ centrifuge d'un séparateur (30, 50 et/ou 80).

4. Procédé selon l'une des revendications précédentes, **caractérisé en ce que** la formation de la suspension de levure selon l'étape B comprend une autolyse (20) des cellules de levure.

5. Procédé selon la revendication 4, **caractérisé en ce que** la formation de la suspension de levure selon l'étape B comprend, après l'autolyse (20), une séparation (30) d'un extrait de levure (31) formant une phase solide (32) ou une phase liquide contenant des solides, la phase solide ou la deuxième phase liquide contenant des composantes solides des cellules de levure et des bêtaglucanes.

6. Procédé selon la revendication 5, **caractérisé en ce que** la formation de la suspension de levure selon l'étape B comprend un ou, de préférence, plusieurs lavages (40) de la phase solide et/ou de la phase liquide contenant des solides.

7. Procédé selon l'une des revendications précédentes, **caractérisé en ce que** la formation de la suspension de levure selon l'étape B comprend une mise en suspension des cellules de levure autolysées.

8. Procédé selon la revendication 7, **caractérisé en ce que** la suspension de levure est traitée avec le nanocavitateur (70, 70' et/ou 70") et/ou **en ce que** le traitement de la suspension de levure avec le nanocavitateur (70‴) a lieu après l'ajout d'une solution alcaline, en particulier d'une lessive (60).

9. Procédé selon l'une des revendications précédentes, **caractérisé en ce qu'**en plus de son traitement dans un nanocavitateur (70, 70', 70" et/ou 70‴), la suspension de levure est traitée dans un homogénéisateur et/ou dans un mélangeur.

10. Procédé selon la revendication 9, **caractérisé en ce que** le traitement supplémentaire de la suspension de levure dans l'homogénéisateur et/ou dans le mélangeur a lieu immédiatement avant ou après son traitement avec le nanocavitateur (70, 70', 70" et/ou 70‴).

11. Procédé selon l'une des revendications précédentes, **caractérisé en ce que** la température de la suspension de levure pendant le traitement dans le nanocavitateur (70, 70', 70" et/ou 70‴) est comprise entre 20 et 90 °C, de préférence entre 50 et 60 °C.

12. Procédé selon l'une des revendications précédentes, **caractérisé en ce que** le traitement dans le nanocavitateur (70, 70', 70" et/ou 70‴) est effectué plusieurs fois.

13. Procédé selon l'une des revendications précédentes, **caractérisé en ce que** le traitement dans le nanocavitateur (70, 70', 70" et/ou 70‴) est effectué à des pressions de 50 à 150 bars, de préférence de 60-90 bars.

14. Procédé selon l'une des revendications précédentes, **caractérisé en ce que** la suspension de levure est mise en circulation dans un circuit qui la fait passer plusieurs fois à travers le nanocavitateur (70, 70', 70" et/ou 70‴) dans un intervalle de temps.

15. Procédé selon l'une des revendications précédentes, **caractérisé en ce que** la multiplication des cellules de levure (10) est effectuée dans une solution sucrée sans exclusion de l'oxygène.

16. Procédé selon l'une des revendications précédentes, **caractérisé en ce qu'**au moins un paramètre de process du traitement avec le nanocavitateur (70, 70', 70" et/ou 70‴), en particulier la durée de traitement, la pression de travail et/ou la température de la suspension de levure, est piloté et/ou régulé à l'aide de la distribution granulométrique des composantes de la levure dans la suspension de levure et/ou de la viscosité dynamique en fonction de la vitesse de cisaillement.

17. Procédé selon l'une des revendications précédentes, **caractérisé en ce que** la suspension de levure obtenue dans l'étape B est disponible et est traitée par le nanocavitateur (70, 70', 70" et/ou 70‴) selon l'étape C
a) après la multiplication des cellules de levure (10) et avant l'autolyse (20),
b) après l'autolyse (20) et avant la séparation de l'extrait de levure (31),
c) lors du lavage (40) et avant la séparation (50) de l'extrait de lavage (51),
et/ou
d) lors de la réaction avec la lessive (60) et avant la séparation (80) de l'extrait de lessive (81).
